Europäisches Patentamt

(19) European Patent Office

Office européen des brevets

(11) Numéro de publication: **0 081 434**

**A1**

(12) # DEMANDE DE BREVET EUROPEEN

(21) Numéro de dépôt: **82402212.3**

(22) Date de dépôt: **03.12.82**

(51) Int. Cl.³: **C 07 C 139/00**
**C 07 C 143/24, E 21 B 43/22**

(30) Priorité: **07.12.81 FR 8122851**

(43) Date de publication de la demande:
**15.06.83 Bulletin 83/24**

(84) Etats contractants désignés:
**FR GB**

(71) Demandeur: **COMPAGNIE FRANCAISE DE RAFFINAGE**
**Société anonyme dite:**
**5, rue Michel-Ange**
**F-75781 Paris Cedex 16(FR)**

(72) Inventeur: **Baradel, Pierpaolo**
**Via Navali 32**
**I-34143 Trieste(IT)**

(72) Inventeur: **Bussi, Giancarlo**
**Strada dei Friuli No 85**
**I-34100 Trieste(IT)**

(72) Inventeur: **Marty, Claude**
**66, rue Guillemard**
**F-76600 Le Havre(FR)**

(74) Mandataire: **Jolly, Jean-Pierre et al,**
**Cabinet BROT et JOLLY 83, rue d'Amsterdam**
**F-75008 Paris(FR)**

(54) **Procédé de préparation de sulfonates d'hydrocarbures aromatiques et application desdits sulfonates à la récupération assistée du pétrole brut.**

(57) Procédé de préparation de sulfonates d'hydrocarbures aromatiques notamment destinés à la récupération assistée de pétrole brut, ce procédé consistant à faire réagir de l'anhydride sulfurique avec une charge comprenant au moins un hydrocarbure aromatique et comportant les étapes suivantes :

a) la réaction de l'anhydride sulfurique avec au moins un hydrocarbure aromatique contenu dans la charge, ladite réaction étant effectuée dans un solvant constitué par un mélange de 5 % à 95 % en volume d'au moins un hydrocarbure halogéné aliphatique ayant de 1 à 6 atomes de carbone et de 95 % à 5 % en volume d'au moins un hydrocarbure aliphatique saturé ayant de 5 à 10 atomes de carbone,

b) la décantation du mélange réactionnel obtenu dans l'étape a), ladite décantation conduisant à l'obtention :

- d'une première phase inférieure, contenant la majorité des acides polysulfoniques obtenus dans l'étape a),
- d'une seconde phase supérieure, contenant la majorité des acides monosulfoniques obtenus dans l'étape a),

c) la neutralisation de la deuxième phase obtenue dans l'étape b),

d) la séparation, à partir de la deuxième phase neutralisée obtenue dans l'étape c), d'un produit contenant la majorité des monosulfonates.

# Procédé de préparation de sulfonates d'hydrocarbures aromatiques et application desdits sulfonates à la récupération assistée du pétrole brut

La présente invention concerne un procédé de préparation de sulfonates à partir d'un mélange d'hydrocarbures aromatiques ou contenant des hydrocarbures aromatiques. Elle concerne plus particulièrement la préparation de sulfonates d'hydrocarbures aromatiques à partir d'un pétrole brut ou d'une fraction de pétrole brut. Elle concerne également l'application de ces sulfonates à la réalisation de microémulsions pour la récupération du pétrole brut contenu dans une formation souterraine -récupération dite "assistée"- par injection dans cette formation de sulfonates, de préférence en compagnie d'agents tensio-actifs, comme les alcools, et/ou d'électrolytes.

Les sulfonates obtenus à partir d'hydrocarbures aromatiques ont de nombreuses applications, notamment comme agents émulsifiants. Ce sont des produits contenant une ou plusieurs fonctions $SO^-_3$, qui sont obtenus par action de l'anhydride sulfurique ou de l'acide sulfurique fumant, l'"oléum", sur un hydrocarbure aromatique contenant un ou plusieurs noyaux aromatiques. Les acides mono- ou polysulfoniques ainsi formés sont ensuite neutralisés par une base d'un métal alcalin ou de l'ammoniaque.

La récupération assistée du pétrole brut a fait l'objet de nombreux brevets. On peut citer par exemple les brevets français n° 2 299 494, 2 321 035 et 2 402 057.

Les sulfonates intéressants pour la récupération assistée doivent comporter dans leur molécule, pour qu'il y ait formation d'une émulsion par l'eau et le pétrole brut, deux groupements ayant des caractères de solubilité totalement différents :

- un groupement soluble dans le pétrole brut ou les solvants (caractère lipophile),

- un groupement soluble dans l'eau et insoluble dans le pétrole brut ou les solvants (caractère hydrophile).

Plus le sulfonate contiendra de fonctions $SO_3^-$ et plus il sera soluble dans l'eau et donc moins apte à aider à la formation d'une émulsion d'un type convenable pour la récupération assistée.

La nature des sulfonates utilisables pour la récupération assistée du pétrole brut dépend naturellement du pétrole et du gisement considérés. La masse moléculaire équivalente moyenne d'un sulfonate étant définie par le rapport

$$\frac{\text{masse moléculaire moyenne du sulfonate}}{\text{nombre moyen de fonctions } SO_3^- \text{ par molécule de sulfonate}}$$

on considère généralement que les sulfonates aptes à servir à la récupération assistée du pétrole brut ont une masse moléculaire équivalente moyenne comprise environ entre 400 et 600.

Autrement dit, si un hydrocarbure aromatique a une masse moléculaire moyenne comprise entre 300 et 550 environ, un sulfonate dérivé de cet hydrocarbure sera intéressant pour la récupération assistée s'il ne contient qu'une fonction $SO_3^-$ (masse égale à 80).

De nombreux brevets ont pour objet des procédés de préparation de sulfonates aptes à la récupération assistée du pétrole brut. Dans ces brevets et dans la littérature, en général, on appelle "sulfonates verts" les mélanges de sulfonates contenant principalement des polysulfonates très hydrophiles, et "sulfates acajous" les mélanges de sulfonates contenant principalement des monosulfonates très lipophiles.

Comme exemple de brevet de préparation de sulfonates à partir d'un mélange d'hydrocarbures, on peut citer le brevet français n° 2 177 728. Ce brevet concerne un procédé de préparation de sulfonates à partir d'un pétrole brut ou d'un pétrole brut étêté (c'est-à-dire, tel que défini dans le brevet, d'un pétrole brut dont la fraction ayant un point d'ébullition inférieur à 315 °C a été enlevée).

La sulfonation de la charge se fait à l'aide de l'anhydride sulfurique, éventuellement dissous dans un solvant de réaction tel que le dichloroéthane. Le courant d'anhydride sulfurique peut être dilué, en même temps que le solvant de réaction, dans un diluant tel qu'une paraffine légère comme le n-pentane. Les exemples de ce brevet ne décrivent toutefois pas l'emploi simultané de dichloroéthane et de n-pentane. La neutralisation des acides sulfoniques formés se fait immédiatement après la sulfonation. Ce brevet n'enseigne donc pas la séparation des "sulfonates verts" et des "sulfonates acajous".

Le brevet français n° 2 010 344 décrit un procédé pour la séparation par décantation d'un mélange d'acides sulfoniques, les acides mono- et polysulfoniques respectivement insolubles et solubles dans l'eau, en ajoutant au mélange un hydrocarbure saturé, tel que l'hexane, avant la décantation.

La Demanderesse a maintenant établi qu'en utilisant un solvant de composition particulière, il est possible de régler la séparation par décantation des acides mono- et polysulfoniques, afin d'obtenir une qualité particulière de sulfonates utiles dans la récupération assistée du pétrole brut.

Le but de la présente invention est donc la mise au point d'un procédé particulièrement sélectif de préparation de sulfonates d'hydrocarbures aromatiques utilisables dans la récupération assistée du pétrole brut.

A cet effet, la présente invention a pour objet un procédé de préparation de sulfonates d'hydrocarbures aromatiques, notamment destinés à la récupération assistée du pétrole brut, ce procédé consistant à faire réagir de l'anhydride sulfurique avec une charge comprenant au moins un hydrocarbure aromatique et étant caractérisé en ce qu'il comporte les étapes successives suivantes :

a/ la réaction de l'anhydride sulfurique avec au moins un hydrocarbure aromatique contenu dans la charge, ladite réaction étant effectuée dans un solvant constitué par un mélange de 5 à 95 % en volume d'au moins un hydrocarbure halogéné aliphatique ayant de 1 à 6 atomes de carbone et de 95 à 5 % en volume d'au moins un hydrocarbure aliphatique saturé ayant de 5 à 10 atomes de carbone,

b/ la décantation du mélange réactionnel obtenu dans l'étape a/, ladite décantation conduisant à l'obtention :

- d'une première phase inférieure, contenant la majorité des acides polysulfoniques obtenus dans l'étape a/,

- d'une seconde phase supérieure, contenant la majorité des acides monosulfoniques obtenus dans l'étape a/,

c/ la neutralisation par une base de la deuxième phase, obtenue dans l'étape b/,

d/ la séparation, à partir de la deuxième phase neutralisée obtenue dans l'étape c/, d'un produit contenant la majorité des monosulfonates.

Un deuxième objet de l'invention est constitué par une variante du procédé défini ci-dessus, dans laquelle la première phase contenant la majorité des acides polysulfoniques est également neutralisée.

Dans le procédé selon l'invention, la charge comprend au moins un hydrocarbure aromatique. Pour des raisons économiques, la charge est, d'une façon générale, constituée d'un mélange d'hydrocarbures contenant plusieurs hydrocarbures mono et/ou polyaromatiques. En effet, il n'est pas réaliste, bien que ce soit théoriquement possible, d'isoler spécialement un hydrocarbure aromatique pour préparer un sulfonate, dans le cas notamment de sulfonates utilisables pour la récupération assistée du pétrole brut.

Les charges utilisables peuvent être notamment constituées par un pétrole brut, ou par un résidu de distillation sous pression atmosphérique du pétrole brut. Il peut également s'agir des différentes fractions obtenues à partir de la distillation sous pression réduite du résidu de distillation sous pression atmosphérique du pétrole brut, et qui sont appelées communément, en allant des fractions ayant un intervalle de température de distillation le plus bas vers celles ayant un intervalle le plus haut, gazole sous vide, distillats 1, 2, 3, 4 et 5 et résidu sous vide.

Il peut également s'agir des produits riches en hydrocarbures aromatiques, obtenus à partir des fractions citées ci-dessus par extraction des hydrocarbures aromatiques à l'aide d'un solvant, comme le furfural.

Des mélanges de différentes charges décrites ci-dessus peuvent être utilisés comme charges de sulfonation.

Comme il a été indiqué précédemment, les charges devront contenir des hydrocarbures aromatiques ayant des masses moléculaires moyennes de préférence comprises entre 300 et 550 environ, bien que des masses comprises entre 200 et 600 environ puissent être envisagées.

Ces charges peuvent ne contenir que des hydrocarbures aromatiques, mais la teneur en hydrocarbures aromatiques des charges est, de préférence, comprise entre 20 et 70 % en poids.

Ces hydrocarbures aromatiques peuvent être notamment constitués par des hydrocarbures ayant de 1 à 6 noyaux aromatiques, sur lesquels peuvent être branchés des restes alkyles, linéaires ou ramifiés, ou des restes cycliques saturés.

L'anhydride sulfurique peut, lors de la réaction, être utilisé à l'état gazeux ou à l'état liquide. Il peut être à l'état gazeux dilué dans un gaz inerte vis-à-vis de lui, comme, par, exemple, de l'azote sec. Dans le cas où il est utilisé à l'état liquide, il est dissous par l'un ou les solvants de la réaction. On peut également utiliser l'anhydride sulfurique contenu dans l'acide sulfurique fumant.

Selon l'invention, le solvant de la réaction est constitué par un mélange de 5 à 95 % en volume d'au moins un hydrocarbure halogéné aliphatique ayant de 1 à 6 atomes de carbone et de 95 à 5 % en volume d'au moins un hydrocarbure aliphatique saturé ayant de 5 à 10 atomes de carbone.

On utilise de préférence de 30 à 70 % en volume de l'un des deux composés et de 70 à 30 % en volume de l'autre composé.

6    **0081434**

La Demanderesse a en effet découvert que l'utilisation de ces gammes de composition du solvant conduisait à l'obtention d'un bon rendement en sulfonates particulièrement adaptés à la récupération assistée du pétrole brut.

La variation de la composition du solvant fait varier les rendements en sulfonates "verts" et en sulfonates "acajous". On peut ainsi adapter le procédé pour obtenir une qualité donnée de sulfonates "acajous", selon la nature du pétrole brut à récupérer.

Lors de la réaction, le pourcentage en poids d'anhydride sulfurique par rapport à la charge peut être compris, par exemple, entre 3 et 20 %, de préférence entre 4 % et 12 % et, mieux encore, entre 7 % et 9 ‰.

La température, lors de la réaction, peut être comprise entre 20 et 80°C et, de préférence, entre 25 et 50°C.

La pression peut être comprise entre $49,05.10^3$ et $981.10^3$ Pa et, de préférence, entre $98,1.10^3$ et $490,5.10^3$ Pa.

Le réacteur est, bien sûr, de préférence réalisé avec un matériau apte à résister à l'action de l'anhydride sulfurique, par exemple en acier inoxydable.

A la sortie du réacteur, le mélange réactionnel est conduit dans un décanteur, après passage éventuel dans un échangeur de température, pour amener le mélange réactionnel à la température désirée.

Dans le décanteur, on obtient une phase inférieure contenant la majorité des polysulfonates et une phase supérieure contenant la majorité des monosulfonates.

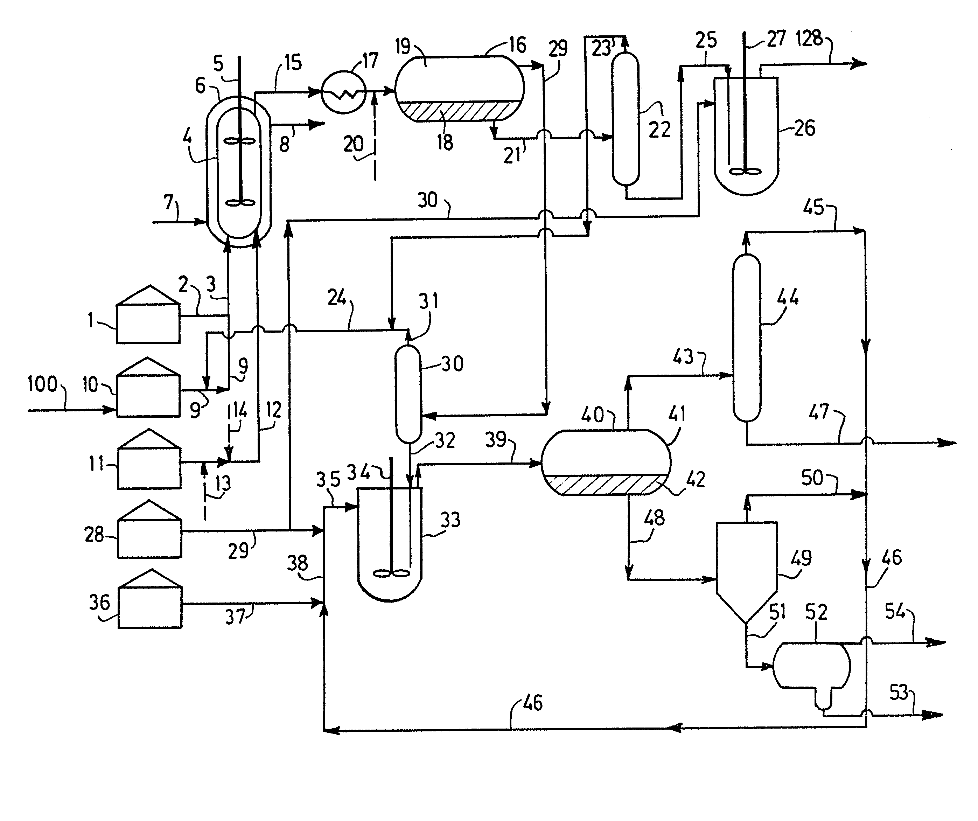

La suite du traitement sera mieux comprise par la description ci-après de la figure unique jointe, sur laquelle est représentée, à titre non limitatif et de façon simplifiée, une unité industrielle mettant en oeuvre le procédé selon l'invention.

On dispose, dans un réservoir 1, d'une charge contenant des hydrocarbures aromatiques. Cette charge est conduite, par les lignes 2 et 3, dans un réacteur de sulfonation 4. Ce réacteur est équipé d'un agitateur 5 et d'une double enveloppe 6, dans laquelle circule un liquide de refroidissement, tel que de l'eau, qui est introduit dans la double enveloppe 6, par la ligne 7, et qui en ressort par la ligne 8. La présence de ce moyen de refroidissement du réacteur est nécessaire pour maintenir la température du réacteur au niveau désiré, car la réaction de sulfonation est exothermique.

Préalablement à son entrée dans le réacteur, on ajoute à la charge, par la ligne 9, un solvant constitué par un mélange de 1,2-dichloroéthane et d'heptane. Par heptane, on entend ici soit du normal heptane, soit de l'heptane ramifié ou un mélange de normal heptane et d'un ou plusieurs heptanes ranifiés. Le solvant provient d'un réservoir 10. Un appoint de solvant peut être introduit dans le réservoir par la ligne 100.

On dispose d'un réservoir 11 contenant de l'anhydride sulfurique. L'anhydride sulfurique peut être utilisé soit à l'état gazeux, soit à l'état liquide. Il est conduit au réacteur de sulfonation par la ligne 12. Dans le cas où il est utilisé à l'état gazeux, il est vaporisé à sa sortie du réservoir 11 dans un vaporisateur non représenté ; on peut le diluer par un gaz inerte tel que de l'azote sec, qui est introduit dans la ligne 12 par la ligne 13. Dans le cas où il est utilisé à l'état liquide, on peut le dissoudre dans un solvant de réaction qui est introduit dans la ligne 12 par la ligne 14.

Les hydrocarbures aromatiques sont sulfonés dans le réacteur 4 et il y a formation d'acides mono- et polysulfoniques. Le mélange réactionnel contenant ces acides, le solvant et les hydrocarbures n'ayant pas réagi, entre autres les hydrocarbures aliphatiques saturés, est conduit par la ligne 15 dans un décanteur 16, après passage éventuel dans un échangeur 17 qui peut être soit un réfrigérant, soit un réchauffeur, selon que l'on désire abaisser ou élever la température du mélange réactionnel avant la décantation. La température dans le décanteur peut être comprise entre 20 et 100°C et la pression entre $98,1.10^3$ et $490,5.10^3$ Pa.

Dans le décanteur 16, le mélange réactionnel se sépare en deux phases. On obtient ainsi une première phase inférieure 18, contenant la majorité des acides polysulfoniques, ainsi qu'une petite quantité d'acides monosulfoniques et du solvant. La deuxième phase supérieure 19 contient la majorité des acides monosulfoniques, ainsi qu'une petite quantité d'acides polysulfoniques et du solvant, et les hydrocarbures n'ayant pas réagi. La séparation dépend des quantités respectives de 1,2-dichloroéthane et d'heptane contenues dans le solvant. Plus la quantité d'heptane sera élevée, plus la quantité de la première phase sera élevée, et moins il y aura d'acides polysulfoniques dans la deuxième phase dont le rendement sera par contre réduit. L'invention permet d'aboutir à un compromis en optimisant les quantités respectives des composés constituant le solvant, d'une part, de façon à avoir un bon rendement en acides monosulfoniques contenant la quantité maximum acceptable d'acides polysulfoniques et, d'autre part, en vue d'obtenir la qualité désirée de sulfonates "acajous" selon la nature du pétrole brut à récupérer. Ces quantités respectives dépendent de la charge traitée, mais la Demanderesse a établi que le solvant pouvait être constitué par un mélange de 5 à 95% en volume de 1,2-dichloroéthane et 95 à 5% en volume d'heptane et, de préférence, de 30 à 70 % en volume de 1,2-dichloroéthane et de 70 à 30% en volume d'heptane. Afin de régler la composition du solvant dans le décanteur, on peut ajouter de l'heptane ou du 1,2-dichloroéthane à l'entrée du décanteur, par la ligne 20.

La première phase inférieure est recueillie dans le fond du décanteur par la ligne 21 et est conduite dans la partie médiane d'une colonne 22 de séparation par détente, fonctionnant à une température qui peut être comprise entre 40 et 150°C et à une pression comprise entre $0,981.10^3$ et $196,2.10^3$ Pa.

On recueille au sommet de la colonne 22, par la ligne 23, le solvant contenu dans la première phase. Ce solvant est recyclé par les lignes 23 et 24 à la ligne 9.

Dans la forme particulière de mise en oeuvre du pro-

cédé selon l'invention représentée sur la figure, on recueille au fond de la colonne 22, par la ligne 25, les acides sulfoniques, en majorité polysulfoniques, qui sont conduits dans une enceinte de neutralisation 26, équipée d'un agitateur 27 et fonctionnant à une température comprise entre 25 et 100°C et à une pression comprise entre $78,5.10^3$ et $196,2.10^3$ Pa. On introduit dans cette enceinte une solution de soude, provenant d'un réservoir 28, et qui est conduite à l'enceinte 26 par les lignes 29 et 30. On recueille à la sortie de l'enceinte 26, par la ligne 128, le mélange de sulfonates constitué en majorité par des polysulfonates appelés "sulfonates verts".

La neutralisation des acides polysulfoniques n'est cependant pas absolument nécessaire dans le procédé selon l'invention.

La deuxième phase, comprenant la majorité des acides monosulfoniques, une petite quantité d'acides polysulfoniques, du solvant et les hydrocarbures non sulfonés, est conduite par la ligne 29 dans la partie médiane d'une colonne 30 de séparation par détente, fonctionnant à une température qui peut être comprise entre 40 et 150°C et à une pression qui peut être comprise entre $0,981.10^3$ et $196,2.10^3$ Pa.

On recueille au sommet de la colonne 30, par la ligne 31, du solvant qui est recyclé à la ligne 9 par la ligne 24.

On recueille au fond de la colonne 30, par la ligne 32, un mélange constitué par la majorité des acides monosulfoniques, une petite quantité d'acides polysulfoniques et les hydrocarbures non sulfonés. Ce mélange est conduit dans une enceinte de neutralisation 33, équipée d'un agitateur 34, fonctionnant à une température comprise entre 25 et 100°C et à une pression comprise entre $78,5.10^3$ et $196,2.10^3$ Pa.

On introduit dans l'enceinte 33 :

- d'une part, par les lignes 29 et 35, une solution de soude provenant du réservoir 28 ;

- d'autre part, par les lignes 37, 38 et 35, une solution aqueuse d'alcool isopropylique provenant d'un réservoir 36, cette solution aqueuse d'alcool isopropylique étant destinée à servir de solvant d'extraction des sulfonates qui vont se former lors de la neutralisation.

On recueille à la sortie de l'enceinte 33, par la ligne 39, un mélange de sulfonates, d'hydrocarbures non sulfonés, d'eau, d'alcool isopropylique et d'autres sels de sodium (sulfates, sulfites, pyrosulfites).

Le mélange est conduit par la ligne 39 dans un décanteur 40, fonctionnant à une température qui peut être comprise entre 25 et 100°C et à une pression qui peut être comprise entre $78,5.10^3$ et $196,2.10^3$ Pa.

Dans le décanteur 40, il y a séparation de deux phases :

- une phase supérieure 41, contenant de l'eau, de l'alcool isopropylique et les hydrocarbures non sulfonés,

- une phase inférieure 42, contenant de l'eau, de l'alcool isopropylique, des sulfonates et les autres sels de sodium.

La phase supérieure est conduite par la ligne 43 dans une colonne de fractionnement 44, fonctionnant à une température comprise entre 40 et 110°C et à une pression comprise entre $0,981.10^3$ et $117,7.10^3$ Pa.

On recueille au sommet de la colonne 44, par la ligne 45, de l'eau et de l'alcool isopropylique, qui sont recyclés à la ligne 37 par l'intermédiaire de la ligne 46.

On recueille au fond de la colonne 44, par la ligne 47, les hydrocarbures non sulfonés.

La phase inférieure 42 du décanteur 40 est conduite par la ligne 48 dans un évaporateur 49, fonctionnant à une température comprise entre 40 et 110°C et à une pression comprise entre $0,981.10^3$ et $117,7.10^3$ Pa.

On recueille au sommet de l'évaporateur 49, par la ligne 50, de l'eau et de l'alcool isopropylique, qui sont recyclés à la ligne 37 par l'intermédiaire de la ligne 46.

On recueille dans le fond de l'évaporateur 49, par la ligne 51, un mélange de sulfonates et d'autres sels de sodium, qui est conduit par la ligne 51 dans un séparateur 52, fonctionnant à une température comprise entre 25 et 80°C et à une pression comprise entre $78,5.10^3$ et $117,7.10^3$ Pa.

On recueille au fond du séparateur 52, par la ligne 53, la majorité des sels de sodium autres que les sulfonates : sulfates, sulfites, pyrosulfites.

On recueille au sommet du séparateur 52, par la ligne 54, un mélange de sulfonates, constitué en majorité par des monosulfonates appelés "sulfonates acajous", mais contenant également un peu d'hydrocarbures non sulfonés et des sels de sodium.

Les exemples qui suivent, et qui n'ont aucun caractère limitatif, concernent la préparation de sulfonates par le procédé selon l'invention et leur application à la réalisation de microémulsions aptes à la récupération assistée de pétrole brut.

### EXEMPLE 1

Cet exemple concerne la préparation de sulfonates "acajous" par le procédé selon l'invention.

On a réalisé différentes préparations à partir d'une même charge en faisant varier la composition du solvant lors des différentes préparations.

### Caractéristiques de la charge

On part d'une charge A, obtenue par distillation à pression atmosphérique d'un pétrole brut MURBAN-KIRKOUK. Le résidu de la distillation à pression atmosphérique est distillé sous pression réduite et la coupe dite "distillat n° 2", dont les caractéristiques sont les suivantes, sert de charge de sulfonation :

- masse moléculaire moyenne : 400,
- % en poids d'hydrocarbures aromatiques : 51.

### Caractéristiques du solvant

Le solvant est constitué d'un heptane industriel et/ou de 1,2-dichloroéthane.

L'heptane industriel a les caractéristiques suivantes :

- masse volumique en g/cm$^3$ à 15 °C :     0,707,
- intervalle de distillation :     92 à 97 °C,
- composition en % en poids :
  - hydrocarbures aliphatiques saturés linéaires principalement à 7 atomes de carbone :     38,
  - hydrocarbures aliphatiques saturés branchés principalement à 7 atomes de carbone et hydrocarbures cycliques branchés :     60,
  - hydrocarbures aromatiques :     2.

Le 1,2-dichloroéthane commercial a les caractéristiques suivantes :

- point d'ébullition :     82 à 83 °C,
- densité à 20 °C, par rapport à l'eau à 4 °C :     1,254.

### Préparation proprement dite des sulfonates

Dans un réacteur de 2 litres, on introduit 800 g de charge et 800 ml de solvant (heptane et/ou 1,2-dichloroéthane en proportions variables).

On fait barboter dans le réacteur un courant d'anhydride sulfurique gazeux dilué dans de l'azote sec à une concentration telle que le courant gazeux contienne environ 10 % en volume d'anhydride sulfurique.

On introduit ainsi 0,5 à 1 mole d'anhydride sulfurique par mole de matière sulfonable (les hydrocarbures aromatiques).

Le réacteur est maintenu à 30 °C.

Après décantation du mélange réactionnel pendant 20 heures à 25°C, on sépare la phase inférieure contenant la majorité des acides polysulfoniques et qui sera appelée ci-après "phase acides polysulfoniques".

On ajoute à la phase supérieure séparée 10 % en volume d'une solution aqueuse à 50 % en volume d'alcool isopropylique. Le mélange obtenu est neutralisé par une solution aqueuse de soude à 50 % en poids.

On ajoute ensuite au mélange neutralisé 10 à 30 % en volume d'une solution aqueuse à 50 % en volume d'alcool isopropylique. On laisse décanter à 50 °C pendant 3 heures.

La phase supérieure contient du solvant, de l'alcool isopropylique et la majorité des hydrocarbures non sulfonés, qui sont séparés en évaporant le solvant et l'alcool isopropylique.

La phase inférieure contient de l'alcool isopropylique, de l'eau, du solvant, les sulfonates et les sels inorganiques et un peu des hydrocarbures non sulfonés.

On sépare l'eau de la phase alcoolique par distillation d'un azéotrope eau - dichloroéthane-alcool isopropylique. La plupart des sels inorganiques restants précipitent et sont séparés par centrifugation. On sépare par évaporation le solvant restant.

On obtient ainsi le sulfonate brut, qui est séché à 100 °C sous pression réduite.

Les résultats des essais effectués en faisant varier la composition du solvant figurent dans le tableau ci-après.

| Essai N° | Composition du solvant | | | | | | | | | |
|---|---|---|---|---|---|---|---|---|---|---|
| | 1 | | 2 | | 3 | | 4 | | 5 | |
| | C7[1] | DCE[2] | C7 | DCE | C7 | DCE | C7 | DCE | C7 | DCE |
| | 0 | 100 | 34 | 66 | 50 | 50 | 66 | 34 | 100 | 0 |
| Rendement en % en poids de sulfonate brut par rapport à la charge | 38 | | 27 | | 21 | | 16 | | 6 | |
| Rendement en % en poids de "phase acides poly-sulfoniques" par rapport à la charge | 9 | | 19 | | 20 | | 22 | | 26 | |
| Rendement en % en poids d'hydrocarbures non sul-fonés par rapport à la charge | 60 | | 66 | | 70 | | 76 | | 80 | |
| % en poids de matière ac-tive[3] dans le sulfonate brut (mesuré selon norme IP 144) | 47 | | 59 | | 65 | | 72 | | 83 | |
| Masse moléculaire équiva-lente moyenne (mesurée selon norme IP 144) | 410 | | 415 | | 425 | | 430 | | 455 | |

(1) C7 = heptane industriel
(2) DCE = 1,2-dichloroéthane
(3) Les matières actives sont les sulfonates de sodium purs. Il reste également dans le sulfonate brut des hydrocarbures non sulfonés et des sels inorganiques de sodium

Les essais 1 et 5 ont été effectués avec des solvants constitués respectivement seulement par du 1,2-dichloeoéthane et par de l'heptane.

On constate, d'après le tableau, que pour les essais 2, 3 et 4 où le solvant est un mélange de 1,2-dichloroéthane et d'heptane, on obtient :

- d'une part, un bon rendement en sulfonates bruts (16 à 27 %), alors qu'avec un solvant constitué d'heptane pur, on obtient seulement 6 %,

- d'autre part, des sulfonates ayant des masses moléculaires équivalentes moyennes suffisantes pour la récupération assistée du pétrole brut.

<u>Evaluation des sulfonates pour la récupération</u>
<u>assistée du pétrole brut</u>

On peut se reporter à ce sujet à l'ouvrage de D.O. SHAH et R.S. SCHECHTER : "Improved oil recovery by surfactant and polymer flooding", p.101 à 118 : "Recent Advances in the Study of low interfacial tensions", par J.C. MORGAN, R.S. SCHECHTER et W.H. WADE, et à l'article de J.L. CAYIAS, R.S. SCHECHTER, W.H. WADE, "The Utilization of Petroleum Sulfonates for producing low Interfacial Tensions between Hydrocarbons and Water", paru dans The Journal of Colloid and Interface Science, 1976.

Pour évaluer la capacité d'un sulfonate donné à la récupération d'un pétrole brut déterminé, on effectue un test avec des hydrocarbures aliphatiques linéaires saturés.

On admet que le sulfonate est satisfaisant s'il forme, avec de tels hydrocarbures ayant de 4 à 12 atomes de carbone, un système d'émulsion triphasique à une température déterminée.

Pour de tels hydrocarbures, un système d'émulsion triphasique est obtenu quand le sulfonate a une masse moléculaire équivalente moyenne allant de 400 à 500 environ.

Dans le cas d'un pétrole brut particulier, on détermine l'hydrocarbure ayant un comportement équivalent au pétrole brut dans les mêmes conditions de température. Le sulfonate est jugé satisfaisant s'il donne un système triphasique avec l'hydrocarbure équivalent.

Un exemple de test consiste à ajouter, à un mélange de 50 % en poids d'eau contenant 5 grammes par litre de chlorure de sodium et de 50 % en poids d'hydrocarbure ou de pétrole brut, 7,5 % en poids de sulfonate et 2,5 % en poids d'alcool isobutylique (en tant qu'agent cotensioactif). On observe le comportement du système réalisé après décantation à température constante.

De tels tests, réalisés avec les sulfonates préparés dans les essais 2, 3 et 4, ont permis d'obtenir des systèmes triphasiques. Ces sulfonates sont donc aptes à la récupération assistée du pétrole brut.

## Exemple 2

Cet exemple concerne la préparation de sulfonates "acajous" par le procédé selon l'invention.

Les essais décrits dans l'exemple 1 ayant montré que l'utilisation d'un solvant constitué par 50 % en volume de 1,2-dichloroéthane et par 50 % en volume d'heptane, permettait d'obtenir des sulfonates "acajous" aptes à la récupération assistée et avec un rendement acceptable, on a effectué des essais en utilisant ce solvant, mais avec d'autres charges.

## Essai N°6

Nature de la charge :

"Distillat 3" obtenu de la même façon que le "Distillat 2", mais ayant un intervalle de point d'ébullition plus élevé.

- Masse moléculaire moyenne :        440,
- % en poids d'hydrocarbures
  aromatiques :                      60.

Préparation :

Identique à celle des essais 1 à 5, à cette différence près que la réaction est effectuée à 40 °C.

Résultats :

- Rendement en % en poids de sulfonate

brut par rapport à la charge :          33,

- Rendement en % en poids de phase
acides polysulfoniques par rapport
à la charge :                           24,

- Rendement en % en poids d'hydrocarbures non sulfonés par rapport à
la charge :                             58,

- % en poids de matière active dans
le sulfonate brut :                     51,

- Masse moléculaire équivalente
moyenne :                               445 .

## Essai N°7

### Nature de la charge :

"Distillat n°4", obtenu de la même façon que les
"Distillat n°2" et "Distillat n°3", mais ayant un intervalle
de point d'ébullition plus élevé.

- Masse moléculaire moyenne :          495,
- % en poids d'hydrocarbures
aromatiques :                          60.

### Préparation :

Identique aux essais précédents, mais à ces différences près que la réaction est effectuée à 40 °C et la décantation pendant 6 heures à 70 °C.

### Résultats :

- Rendement en % en poids de sulfonate
brut par rapport à la charge :         22,

- Rendement en % en poids de phase
acides polysulfoniques par rapport
à la charge :                          17,

- Rendement en % en poids d'hydrocarbures non sulfonés par rapport
à la charge :                          71,

- % en poids de matière active dans
le sulfonate brut :                    63,

- Masse moléculaire équivalente
moyenne :                              510.

### Essai N°8

Nature de la charge :

La charge est un mélange à 50 % en volume de "Distillat n°3" et 50 % en volume de "Distillat n°4".

Préparation :

Identique à celle de l'essai n°7.

Résultats :

- Rendement en % en poids de sulfonate
  brut par rapport à la charge :                    17,
- Rendement en % en poids de phase acides
  sulfoniques par rapport à la charge :             17,
- Rendement en % en poids d'hydrocarbures
  non sulfonés par rapport à la charge :      73,
- Masse moléculaire équivalente moyenne :   495.

Les résultats des essais 6 à 8 montrent que l'on obtient, avec des rendements acceptables, des sulfonates ayant des masses moléculaires équivalentes moyennes souhaitées pour la récupération assistée du pétrole brut.

Des essais de réalisation de microémulsions tels que décrits dans l'exemple 1 ont été positifs.

REVENDICATIONS

1 - Procédé de préparation de sulfonates d'hydrocarbures aromatiques notamment destinés à la récupération assistée de pétrole brut, ce procédé consistant à faire réagir de l'anhydride sulfurique avec une charge comprenant au moins un hydrocarbure aromatique et étant caractérisé en ce qu'il comporte les étapes suivantes :

a) la réaction de l'anhydride sulfurique avec au moins un hydrocarbure aromatique contenu dans la charge, ladite réaction étant effectuée dans un solvant constitué par un mélange de 5 % à 95 % en volume d'au moins un hydrocarbure halogéné aliphatique ayant de 1 à 6 atomes de carbone et de 95 % à 5 % en volume d'au moins un hydrocarbure aliphatique saturé ayant de 5 à 10 atomes de carbone,

b) la décantation du mélange réactionnel obtenu dans l'étape a), ladite décantation conduisant à l'obtention :

- d'une première phase inférieure, contenant la majorité des acides polysulfoniques obtenus dans l'étape a),
- d'une seconde phase supérieure, contenant la majorité des acides monosulfoniques obtenus dans l'étape a),

c) la neutralisation de la deuxième phase obtenue dans l'étape b),

d) la séparation, à partir de la deuxième phase neutralisée obtenue dans l'étape c), d'un produit contenant la majorité des monosulfonates.

2 - Procédé selon la revendication 1, caractérisé en ce que le solvant est constitué de 30 à 70 % en volume d'au moins un hydrocarbure aliphatique halogéné et de 70 à 30 % en volume d'au moins un hydrocarbure aliphatique saturé.

3 - Procédé selon la revendication 1 ou 2, caractérisé en ce que le solvant est constitué par un mélange de 1,2-dichloroéthane et d'heptanes.

4 - Procédé selon l'une des revendications 1 à 3, caractérisé en ce que la première phase inférieure obtenue

0081434

dans l'étape b) est également neutralisée.

5 - Les sulfonates "acajous" obtenus par un procédé selon l'une des revendications 1 à 4.

6 - Application des sulfonates selon la revendication 5 à la récupération assistée du pétrole brut.

Office européen
des brevets

**RAPPORT DE RECHERCHE EUROPEENNE**

EP 82 40 2212

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A,D | --- <br> FR-A-2 010 344 (CONTINENTAL OIL COMPANY) <br> *En entier* | 1 | C 07 C 139/00 <br> C 07 C 143/24 <br> E 21 B 43/22 |
| A,D | --- <br> FR-A-2 177 728 (MARATHON OIL COMPANY) <br> *En entier* <br><br> ----- | 1 | |

| | | | DOMAINES TECHNIQUES RECHERCHES (Int. Cl. 3) |
|---|---|---|---|
| | | | C 07 C 143/00 |

Le présent rapport de recherche a été établi pour toutes les revendications

| Lieu de la recherche | Date d'achèvement de la recherche | Examinateur |
|---|---|---|
| LA HAYE | 17-02-1983 | VAN GEYT J.J.A. |

OEB Form 1503. 03.82